# EUROPÄISCHE PATENTSCHRIFT

(11) **EP 4 110 224 B1**
(45) Veröffentlichungstag und Bekanntmachung des Hinweises auf die Patenterteilung: **06.12.2023**
(21) Anmeldenummer: 22713607.4
(22) Anmeldetag: 08.03.2022
(51) Int. Cl.: A61B 50/20, A61B 50/33, A61B 50/34

(54) **AUFNAHME-EXPANDERHÜLSE SOWIE WERKZEUGSET**
EXPANDING HOLDER AND TOOL SET
DOUILLE EXPANSIBLE DE RÉCEPTION ET JEU D'INSTRUMENTS

(30) Priorität: 12.03.2021 DE 102021106110
(43) Veröffentlichungstag der Anmeldung: 04.01.2023
(73) Patentinhaber: Aesculap AG, 78532 Tuttlingen (DE)
(72) Erfinder: AY, Eda, 88512 Mengen (DE); KNITTEL, Timo, 78573 Wurmlingen (DE)
(74) Vertreter: Winter, Brandl - Partnerschaft mbB
(86) Internationale Anmeldenummer: PCT/EP2022/055882
(87) Internationale Veröffentlichungsnummer: WO 2022/189427

(56) Entgegenhaltungen:
- EP-A1- 0 263 011
- EP-A2- 0 916 406
- WO-A1-2020/078745
- US-A1- 2010 001 152
- US-A1- 2017 143 449

## Beschreibung

### Technisches Gebiet

Die vorliegende Offenbarung betrifft ein Werkzeugset mit einer Aufnahme-Expanderhülse zur steckgelagerten Aufnahme chirurgischer Werkzeuge und einer Halterungsvorrichtung.

### Hintergrund der Erfindung

In der modernen Chirurgie kommt eine Vielzahl von verschiedenen Instrumentenköpfen/Werkzeugen wie z.B. Bohr- oder Fräswerkzeuge (mit Werkzeugschaft) zum Einsatz, die jeweils auf/in ein einen Instrumentenantrieb/Motor aufweisendes Handstück abnehmbar auf-/eingesteckt werden können. Dies erlaubt dem Operateur, während des Betriebs des Handstücks den auf dem / in das Handstück auf-/eingesteckten Instrumentenschaft mit distalem Instrumentenkopf/Effektor je nach Anforderung der Anwendung zu wechseln. Um dem Operateur hierbei einen möglichst einfachen Zugriff auf diese Instrumentenköpfe (nachstehend nur noch als Werkzeuge bezeichnet) zu ermöglichen, werden die Werkzeuge gewöhnlich in einem chirurgischen Werkzeugset geordnet aufbewahrt, wie dies beispielsweise auch aus dem Handwerk beispielsweise für Bohrersets bekannt ist. Hierbei werden, um Beschädigungen der Werkzeuge zu vermeiden und ein rasches Auffinden eines benötigten Werkzeugs zu gewährleisten, die Werkzeuge jedoch nicht lose aufbewahrt, sondern in Aufnahme-Expanderhülsen in fixierender/eingespannter Weise aufgenommen, die wiederum in einer Lochmatrix des Werkzeugsets (im einfachsten Fall eine Lochplatte) eingesteckt bzw. darin fixiert sind.

Auf diese Weise sind die einzelnen Werkzeug coaxial/parallelbeabstandet aneinandergereiht.

### Stand der Technik

Herkömmliche Werkzeugsets dieser Art für die Lagerung/Aufbewahrung von chirurgischen Werkzeugen gehören zum allgemeinen Stand der Technik. Diese bekannten Werkzeugsets weisen im Wesentlichen eine erste Lochplatte (ein sogenanntes Hülsenblech) auf, das als eine üblicherweise metallische Platte mit einer Lochmatrix aus runden Löchern unterschiedlicher Größe ausgebildet ist. In die Löcher sind Aufnahme-Expanderhülsen für die Aufnahme von Werkzeugen eingesteckt bzw. darin fixiert. Die Aufnahme-Expanderhülsen weisen jeweils eine Einstecköffnung für ein bestimmtes chirurgisches Werkzeug auf. Chirurgische Werkzeuge haben einen distalen Werkzeugeingriffsabschnitt und einen proximalen Schaftabschnitt, wobei Aufnahme-Expanderhülsen mit verschiedenen Durchmessern der Werkzeugaufnahmeöffnungen die korrekte und sitzgenaue Aufnahme von Werkzeugen mit verschiedenen Schaftdurchmessern erlauben. Zusätzlich kann das Werkzeugset eine mit der ersten Lochplatte verbundene zweite Lochplatte (ein sogenanntes Bodenblech) aufweisen, das parallel der ersten Lochplatte beabstandet ist und eine Einstecktiefe der chirurgischen Werkzeuge begrenzt.

Neben der schonenden Lagerung chirurgischer Werkzeuge, ist die gründliche Reinigung und Sterilisation chirurgischer Werkzeuge von größter Wichtigkeit. Herkömmliche Aufnahme-Expanderhülsen sind als eine massive Kunststoffhülse mit elastisch radial-expandierbarem Inlay ausgebildet, wodurch das darin eingesteckte Werkzeug zwar sicher gehalten, jedoch eine ausreichende Umströmung der darin aufbewahrten Werkzeuge mit Reinigungsflüssigkeiten oder -gasen erschwert oder gar verhindert wird.

Zur Verbesserung der Umströmung der Werkzeuge ist beispielsweise aus der EP 3 164 095 B1 eine Aufnahme-Expanderhülse bekannt, die einen mit radialen Durchbrüchen versehenen Hohlkörper aus Kunststoff sowie innerhalb des Hohlkörpers eingesetzte, nach innen ragende filigrane Klemmarme vorzugsweise aus Kunststoff oder Metall aufweist. Dabei dient der vergleichsweise massive Kunststoffhohlkörper zum Befestigen der Aufnahme-Expanderhülse an einer eine Lochplatte aufweisenden Haltungsvorrichtung, wohingegen die filigranen Klemmarme zum punktartigen Kontaktieren des in die Aufnahme-Expanderhülse eingesteckten Werkzeugs mit möglichst geringer Kontaktfläche dienen.

Zwar zeichnen sich diese bekannten Aufnahme-Expanderhülsen durch eine hohe Stabilität aus, haben jedoch auch weiterhin den Nachteil, dass der Kunststoffhohlkörper zwar radiale Durchbrüche aufweist, um das Eindringen und Umspülen des Reinigungsfluids zu ermöglichen, es aber immer noch möglich ist, dass sich beispielsweise Gewebereste und dergleichen Verunreinigungen an der Aufnahme-Expanderhülse sammeln können.

Ein weiterer entscheidender Nachteil an diesen bekannten Aufnahme-Expanderhülsen liegt darin, dass die Kunststoffhohlkörper in der Herstellung, Montage und Bestückung teuer und aufwändig sind. Um hohen Temperaturen bei der Sterilisation widerstehen zu können, müssen die Kunststoffteile aus speziellem temperaturresistentem Kunststoff gefertigt sein, was die Fertigungskosten erhöht. Um diese medizinische Anforderung erfüllen zu können, wird oftmals der Werkstoff PEEK verwendet, der als solcher kostenintensiv und schwer zu verarbeiten ist sowie eine begrenzte Lebensdauer aufweist. Zudem ist es bei einem mehrteiligen Aufbau der Aufnahme-Expanderhülse, insbesondere mit dem Kunststoffhohlkörper und den darin eingelassenen oder eingesetzten Metallklemmarmen, erforderlich, die einzelnen Bestandteile in manueller Handarbeit zusammenzuführen. Dies ist nicht nur zeitaufwändig, sondern birgt auch das Risiko der Beschädigung der Aufnahme-Expanderhülsen oder der fehlerhaften Zusammenführung bei der Montage. Zudem bleiben Verschmutzungen an Kunststoff aufgrund dessen positiver Oberflächenladung oft länger haften als an anderen Werkstoffen wie beispielsweise Metall, was die Reinigung der Aufnahme-Expanderhülse zusätzlich erschwert. Auch bei der Trocknung des Werkzeugsets nach der Reinigung erweist sich der massive Aufbau herkömmlicher Werkzeugsets und deren Fertigungsmaterial Kunststoff als nachteilig.

Die in den Kunststoffhohlkörper eingesetzten Metallklemmarme der aus dem Stand der Technik bekannten Werkzeugsets und Aufnahme-Expanderhülsen lassen sich jedoch ohne die Kunststoffhohlkörper nicht oder nicht stabil an der Halterungsvorrichtung der bekannten Werkzeugsets befestigen.

Weiter sind aus der US 2010/001152 A1, EP 0 916 406 A2 oder WO 2020/078745 A1 weitere Metallklammern zum Aufnehmen von Werkzeugen bekannt.

Es ist also die Aufgabe der vorliegenden Offenbarung, eine Aufnahme-Expanderhülse eines Werkzeugsets sowie ein Werkzeugset bereitzustellen, welche einfach und kostengünstig herzustellen sind, eine effiziente Reinigung und/oder Sterilisation gewährleisten sowie eine einfach aufgebaute und gleichzeitig stabile Befestigung der Aufnahme-Expanderhülse an einer Halterungsvorrichtung des Werkzeugs ermöglichen.

Die Aufgabe der vorliegenden Offenbarung wird durch ein Werkzeugset mit den Merkmalen des unabhängigen Patentanspruchs gelöst. Vorteilhafte Weiterbildungen sind Gegenstand der Unteransprüche.

Der Kern der Offenbarung besteht demzufolge darin, dass die Aufnahme-Expanderhülse neben den Klemmarmen zum Halten eines einzusteckenden Werkzeugs den plattenförmigen und abgesehen von den Klemmarmen in Axialrichtung abkantfreien Auffangboden besitzt, der zum unmittelbaren stoffschlüssigen Befestigen an der Halterungsvorrichtung in seinem Mittenbereich zumindest einer seiner Flachseiten einen vorzugsweise metallischen, insbesondere schweißbaren, Anschweiß-, Löt- oder Klebabschnitt ausbildet. Zudem zeichnet sich die Halterungsvorrichtung des Werkzeugsets dadurch aus, dass im Gegensatz zu den Halterungsvorrichtungen des Stands der Technik die Löcher zweier miteinander beabstandet verbundener Lochplatten versetzt zueinander angeordnet sind, so dass die Löcher einer ersten Lochplatte zum Einstecken der Werkzeuge mit Material zwischen den Löchern der zweiten Lochplatte fluchten, um den Auffangboden an dem Material der zweiten Lochplatte stoffschlüssig, insbesondere durch Schweißen, anbringen zu können.

Genauer gesagt dient die Aufnahme-Expanderhülse zur steckgelagerten, insbesondere senkrechten, Aufnahme chirurgischer Werkzeuge. Die Aufnahme-Expanderhülse weist eine Anzahl von umfangsbeabstandeten, elastisch verformbaren Klemmarmen auf, die an einer offenen Stirnseite der Aufnahme-Expanderhülse eine Einstecköffnung für das Einstecken eines Werkzeugs ausbilden und radial nach innen ragende Eingriffsabschnitte zur kraft- und/oder formschlüssigen Kontaktierung des Werkzeugs für das Halten des Werkzeugs haben. Die Klemmarme können vorzugsweise aus Metall gefertigt sein und in Umfangsrichtung der Aufnahme-Expanderhülse gleichverteilt angeordnet sein. Zudem weist die Aufnahme-Expanderhülse einen vorzugsweise metallischen Auffangboden auf, der an einem der Einstecköffnung gegenüberliegenden Endabschnitt der Aufnahme-Expanderhülse ausgebildet ist und von dem sich die Klemmarme aus in Axialrichtung zu der offenen Stirnseite hin erstrecken. Vorzugsweise können die Klemmarme an dem Auffangboden angeformt sein. Durch den Auffangboden kann eine Einstecktiefe des einzusteckenden Werkzeugs begrenzt werden.

Gemäß einem Aspekt der Offenbarung ist der Auffangboden der Aufnahme-Expanderhülse zur unmittelbaren stoffschlüssigen Befestigung der Aufnahme-Expanderhülse an einer vorzugsweise eine Lochplatte aufweisenden Halterungsvorrichtung des Werkzeugsets durch eine vorzugsweise ebene oder gewölbte abkantfreie Platte gebildet, von der sich ausschließlich die Klemmarme axial, d.h. in einer Richtung senkrecht zu der Platte, wegerstrecken und die in einem Mittenbereich an ihrer einen oder beiden Flachseiten einen Anschweiß-, Löt- oder Klebabschnitt ausbildet. Das heißt also, dass von dem Auffangboden ausschließlich die Klemmarme abstehen, so dass der Auffangboden flächig an die Halterungsvorrichtung angelegt und daran befestigt werden kann. Dies hat den Vorteil, dass keine separate oder an der Aufnahme-Expanderhülse ausgebildete Befestigungsvorrichtung ausgebildet sein muss, so dass eine Montage der Befestigungsvorrichtung an den Klemmarmen entfallen kann, aber gleichzeitig eine sichere und stabile Befestigung der Aufnahme-Expanderhülse durch Stoffschluss über den an dem Auffangboden ausgebildeten Anschweiß-, Löt- oder Klebabschnitt gewährleistet ist.

Gemäß einer bevorzugten Ausführungsform können/kann eine von der Einstecköffnung abgewandte Unterseite des Auffangbodens und/oder eine der Einstecköffnung zugewandte Oberseite des Auffangbodens den vorzugsweise metallischen Anschweiß-, Löt- oder Klebabschnitt ausbilden, der zur unmittelbaren stoffschlüssigen, insbesondere (punkt-)schweißenden, Befestigung der Aufnahme-Expanderhülse an der Halterungsvorrichtung des Werkzeugsets angepasst und vorgesehen ist. Bei einer Ausbildung der Anschweiß-, Löt- oder Klebabschnitt an der Unterseite kann die Aufnahme-Expanderhülse in einfacher Weise axial von oben (in der Einsteckrichtung des einzusteckenden Werkzeugs) auf die Halterungsvorrichtung aufgesetzt/aufgelegt und stoffschlüssig daran befestigt werden. Bei einer Ausbildung der Anschweiß-, Löt- oder Klebabschnitt an der Oberseite kann die Aufnahme-Expanderhülse in einfacher Weise axial von unten (entgegen der Einsteckrichtung des einzusteckenden Werkzeugs) auf die Halterungsvorrichtung aufgesteckt und stoffschlüssig daran befestigt werden.

Gemäß einer bevorzugten Ausführungsform kann die Aufnahme-Expanderhülse derart angepasst und vorgesehen sein, dass sie auf die Löcher aufweisende Lochplatte der Halterungsvorrichtung entgegen einer Einsteckrichtung des einzusteckenden Werkzeugs aufsteckbar ist, so dass die Klemmarme durch die Löcher hindurchgreifen und der Auffangboden axial von der Lochplatte als Abstandshalter hervorsteht. Die Löcher können vorzugsweise rund oder sternförmig ausgebildet sein. Mit anderen Worten kann die Lochplatte dadurch beispielsweise gegenüber einem Aufnahmekasten, in den die Lochplatte in der Einsteckrichtung des Werkzeugs eingesetzt wird, beabstandet gehalten werden. Das heißt, dass eine Unterseite der Lochplatte um die Dicke des Auffangbodens von dem Aufnahmekasten axial beabstandet gehalten werden kann, so dass eine Umspülung mit Reinigungsfluid verbessert wird.

Gemäß einer bevorzugten Ausführungsform kann der Auffangboden einen schweißbaren Einsatz (/Inlay) oder eine schweißbare Beschichtung aufweisen. Der schweißbare Einsatz kann beispielsweise als kreisförmige oder ringförmige Platte in dem Mittenbereich des Auffangbodens integriert sein. Dies hat den Vorteil, dass der Auffangboden bzw. die Aufnahme-Expanderhülse als Ganzes aus einem nichtschweißbaren Material ausgebildet werden kann und trotzdem eine schweißbare Anbringung der Aufnahme-Expanderhülse ermöglicht wird.

Gemäß einer vorteilhaften Weiterbildung der bevorzugten Ausführungsform können die Klemmarme und der Auffangboden einstückig aus einem biegeelastischen Metall, vorzugsweise einem Federstahl, beispielsweise aus dem Werkstoff 1.4310, ausgebildet sein. Durch die metallische Ausbildung kann die Aufnahme-Expanderhülse besonders effizient gereinigt und sterilisiert werden.

Gemäß einer bevorzugten Ausführungsform kann die Aufnahme-Expanderhülse als ein Biegeumformteil ausgebildet sein. Dadurch kann die Aufnahme-Expanderhülse als Ganzes besonders einfach und kostengünstig vorzugsweise aus einem metallischen Werkstoff hergestellt werden. Durch die einstückige Ausbildung sind keine zusätzlichen Montageschritte zum Zusammenbau mehrerer Einzelteile notwendig.

Genauer gesagt dient das Werkzeugset zur steckgelagerten Aufnahme chirurgischer Werkzeuge. Das Werkzeugset weist die oben beschriebene Aufnahme-Expanderhülse sowie eine Halterungsvorrichtung auf, die eine erste Lochplatte mit ersten vorzugsweise runden Löchern zum Einstecken eines Werkzeugs sowie eine parallel dazu beabstandete, mit der ersten Lochplatte verbundene zweite Lochplatte mit voneinander durch zweite Stege getrennten zweiten Löchern aufweist. Dabei überschneiden sich die zweiten Stege an mit den ersten Löchern fluchtend angeordneten Kreuzungspunkten, und der Auffangboden der Aufnahme-Expanderhülse ist an den Kreuzungspunkten der zweiten Stege stoffschlüssig befestigt oder befestigbar, insbesondere angeschweißt oder anschweißbar. Dadurch, dass die ersten Löcher zu den zweiten Stegen fluchtend angeordnet sind, bildet sich eine geeignete Auflagefläche und stoffschlüssige Befestigungsfläche für den Auffangboden, wenn die Aufnahme-Expanderhülse durch die ersten Löcher in der Einsteckrichtung des einzusteckenden Werkzeugs in die Halterungsvorrichtung eingesetzt wird.

Gemäß einer bevorzugten Ausführungsform können die zweiten Stege an den Kreuzungspunkten eine Schweiß-, Löt- oder Klebfläche bilden, die zumindest so groß wie eine Fläche des Auffangbodens, insbesondere wie der Anschweiß-, Löt- oder Klebabschnitt des Auffangbodens ist. Dadurch wird gewährleistet, dass die Aufnahme-Expanderhülse (groß-)flächig an der zweiten Lochplatte befestigt oder befestigbar sind.

Gemäß einer bevorzugten Ausführungsform können die zweiten Löcher im Wesentlichen rechteckig, insbesondere quadratisch ausgebildet und in 60° versetzten Reihen angeordnet sein. Das heißt, dass die zweiten Löcher durch die sich an den Kreuzungspunkten T-förmig schneidenden zweiten Stege voneinander getrennt sind. Gemäß der bevorzugten Ausführungsform können sich die zweiten Stege derart verbreitern, dass sie sich an den Eckpunkten der zweiten Löcher vorzugsweise konvex in Richtung zur Lochmitte hin wölben. Das heißt, dass an den Kreuzungspunkten zweier Stege (viertelkreisförmiges) Material der Lochplatte in dem durch die Stege eingeschlossenen Winkel vorhanden ist, das den Querschnitt der zweiten Löcher verringert. Dadurch ergibt sich eine im Wesentlichen quadratische Form der zweiten Löcher, deren Eckpunkte sich etwa viertelkreisförmig nach innen wölben. Dies hat zum einen den Vorteil, dass eine ausreichend große Fläche für das stoffschlüssige Befestigen der Aufnahme-Expanderhülse an den Kreuzungspunkten gebildet wird. Durch die mittig zueinander versetzten zweiten Löcher benachbarter Reihen ergibt sich zudem, dass mehrere Aufnahme-Expanderhülse platzsparend aber mit genügend Abstand zueinander an der zweiten Lochplatte angebracht werden können.

Gemäß einer bevorzugten Ausführungsform kann die Unterseite des Auffangbodens Aufnahme-Expanderhülse an einer der ersten Lochplatte zugewandten Axialseite der zweiten Lochplatte stoffschlüssig befestigt oder befestigbar, insbesondere angeschweißt oder anschweißbar sein. So kann die Aufnahme-Expanderhülse in einfacher Weise axial von oben (in der Einsteckrichtung des einzusteckenden Werkzeugs) auf die Halterungsvorrichtung aufgesetzt/aufgelegt und stoffschlüssig daran befestigt werden. Alternativ oder zusätzlich kann die Oberseite des Auffangbodens an einer der ersten Lochplatte angewandten Axialseite der zweiten Lochplatte stoffschlüssig befestigt oder befestigbar, insbesondere angeschweißt oder anschweißbar sein. So kann in einfacher Weise die Aufnahme-Expanderhülse axial von unten (entgegen der Einsteckrichtung des einzusteckenden Werkzeugs) auf die Halterungsvorrichtung aufgesteckt und stoffschlüssig daran befestigt werden.

Gemäß der bevorzugten Ausführungsform kann die Aufnahme-Expanderhülse, mit ihren Klemmarmen durch die zweiten Löcher hindurchgreifend, entgegen der Einsteckrichtung des einzusteckenden Werkzeugs auf einen der Kreuzungspunkte so aufsteckbar sein, dass der Auffangboden axial von der zweiten Lochplatte als axialer Abstandshalter hervorsteht. Mit anderen Worten kann die zweite Lochplatte dadurch beispielsweise gegenüber einem Aufnahmekasten, in den die zweite Lochplatte in der Einsteckrichtung des Werkzeugs eingesetzt wird, beabstandet gehalten werden. Das heißt, dass eine Unterseite der zweiten Lochplatte um die Dicke des Auffangbodens von dem Aufnahmekasten axial beabstandet gehalten werden kann, so dass eine Umspülung mit Reinigungsfluid verbessert wird.

Gemäß einer bevorzugten Ausführungsform können die ersten Löcher der ersten Lochplatte in parallel beabstandeten Reihen angeordnet sein und die Lochmitten jeweils zwei benachbarter Reihen um eine Stegbreite der zweiten Stege zueinander versetzt angeordnet sein. Dadurch wird eine fluchtende Anordnung der ersten Löcher mit den Kreuzungspunkten und damit mit den Aufnahme-Expanderhülsen gewährleistet. So können mehrere Aufnahme-Expanderhülse (und somit mehrere Werkzeuge) platzsparend aber mit genügend Abstand zueinander an der Halterungsvorrichtung befestigt werden.

Mit anderen Worten betrifft die Offenbarung eine angeschweißte Klemmfeder, bei der eine ein einzusteckendes Werkzeug elastisch, d.h. federnd, lagernde Metallklammer mittels stoffschlüssiger, insbesondere (punkt-)schweißender Befestigung unterhalb (von unten) oder oberhalb (von oben) an einer zweiten Lochplatte einer Halterungsvorrichtung, die beabstandet mit einer ersten Lochplatte einer Halterungsvorrichtung mit ersten Löchern zum Einstecken des Werkzeugs, angebracht werden kann. Eine Form der ersten Löcher kann die Lage des Werkzeugs stabilisieren und/oder durch unterschiedliche Innendurchmesser die Werkzeugschaftdurchmesser eingrenzen. Eine Form von zweiten Löchern der zweiten Lochplatte bzw. ein Abstand der zweiten Lochplatte kann eine Einstecktiefe der Werkzeuge eingrenzen.

### Kurzbeschreibung der Figuren

Fign. 1 bis 23 zeigen verschiedene Ansichten und Modifikationen einer form- und kraftschlüssig befestigbaren Aufnahme-Expanderhülse sowie eines Werkzeugsets mit der Aufnahme-Expanderhülse gemäß einem Aspekt der Offenbarung.
Fign. 24 bis 26 zeigen verschiedene Ansichten und Modifikationen einer formschlüssig einhängbaren Aufnahme-Expanderhülse sowie eines Werkzeugsets mit der Aufnahme-Expanderhülse gemäß einem anderen Aspekt der Offenbarung.
Fign. 27 bis 38 zeigen verschiedene Ansichten und Modifikationen einer stoffschlüssig befestigbaren Aufnahme-Expanderhülse sowie eines Werkzeugsets mit der Aufnahme-Expanderhülse gemäß einem weiteren Aspekt der Offenbarung.

### Beschreibung der Ausführungsformen

Nachstehend werden Ausführungsformen der vorliegenden Offenbarung auf der Basis der zugehörigen Figuren beschrieben.

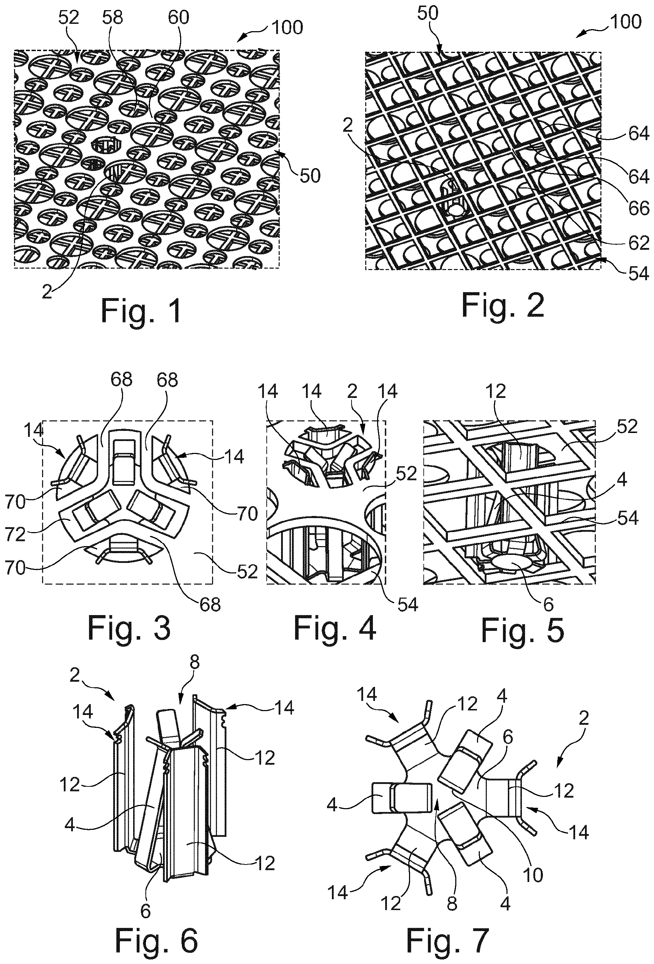

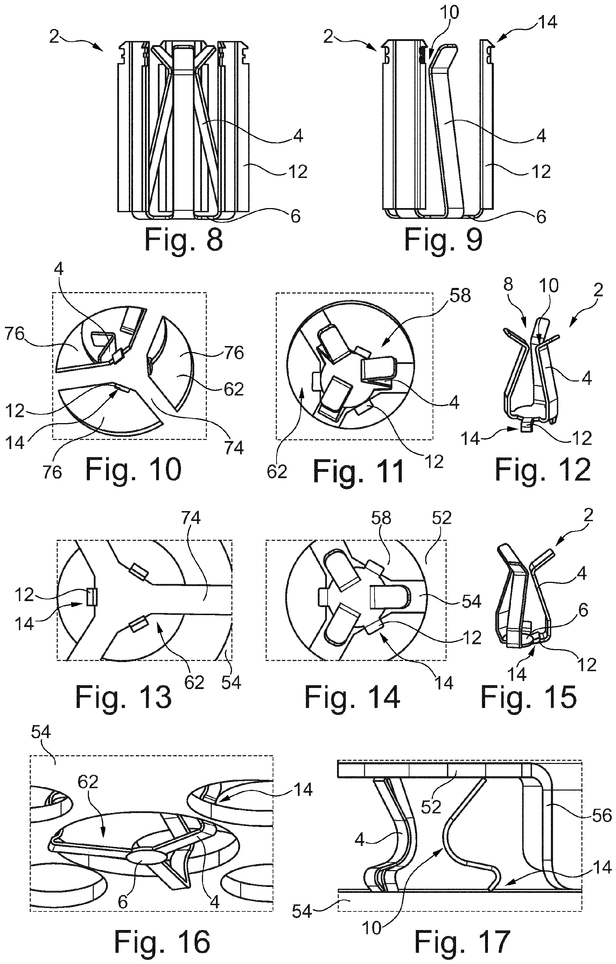

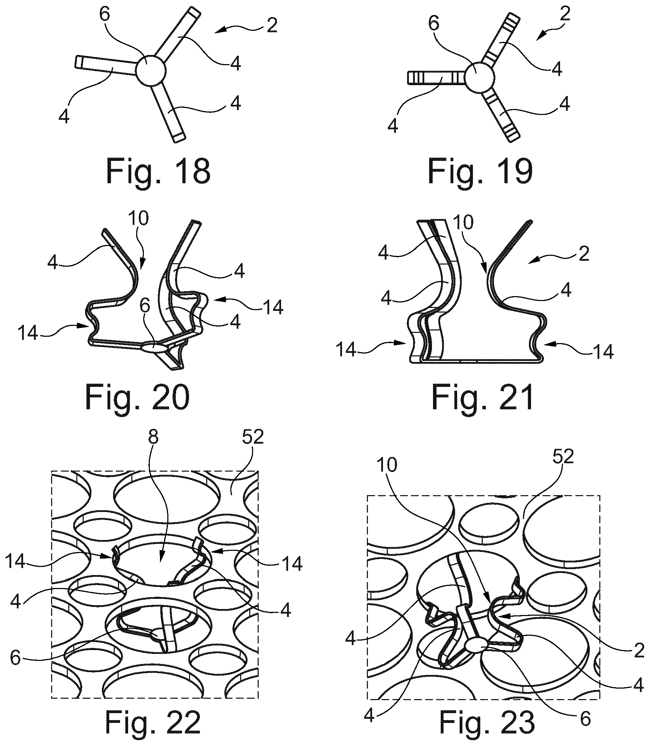

Fign. 1 bis 23 zeigen eine erste Ausführungsform der vorliegenden Offenbarung in verschiedenen Modifikationen. Fign. 1 bis 9 zeigen eine erste Modifikation der ersten Ausführungsform. Fign. 10 bis 12 zeigen eine zweite Modifikation der ersten Ausführungsform. Fign. 13 bis 15 zeigen eine dritte Modifikation der ersten Ausführungsform. Fing. 16 bis 21 zeigen eine vierte Modifikation der ersten Ausführungsform. Fign. 22 und 23 zeigen eine fünfte Modifikation der ersten Ausführungsform.

Fign. 1 bis 5 zeigen eine Aufnahme-Expanderhülse 2 eines Werkzeugsets 100, die in eine Halterungsvorrichtung 50 des Werkzeugsets 100 eingesetzt bzw. eingesteckt ist. Fign. 6 bis 9 zeigen eine Darstellung der Aufnahme-Expanderhülse 2, die nicht in der Halterungsvorrichtung 50 des Werkzeugsets 100 eingesteckt bzw. eingesetzt ist.

Die Aufnahme-Expanderhülse 2 hat eine Anzahl von umfangsbeabstandeten Klemmarmen 4 und einen vorzugsweise ebenen Auffangboden 6, von dem sich die Klemmarme 4 aus in Axialrichtung, d.h. senkrecht zu dem Auffangboden 6, erstrecken. Alternativ kann der Auffangboden 6 auch gewölbt sein oder eine Prägung, einen Überstand oder dergleichen aufweisen, auch wenn dies nicht dargestellt ist. In der dargestellten Ausführungsform hat die Aufnahme-Expanderhülse 2 drei Klemmarme 4. Alternativ könnte die Aufnahme-Expanderhülse 2 auch mehr als 3, beispielsweise 4, 5 oder 6 Klemmarme 4 haben. Die Klemmarme 4 sind elastisch verformbar und bilden an einer offenen Stirnseite der Aufnahme-Expanderhülse 2 eine Einstecköffnung 8 für das Einstecken eines (nicht dargestellten) Werkzeugs aus. Die Klemmarme 4 können gleichverteilt über den Umfang der Aufnahme-Expanderhülse 2 angeordnet sein. Vorzugsweise sind die Klemmarme 4 biegeelastisch und aus einem Metall, beispielsweise aus einem Federstahl, wie dem Werkstoff 1.4310, gefertigt. Die Klemmarme 4 bilden an ihren (stirnseitigen) freien Endabschnitten vorzugsweise einen Trichter, der sich in Einsteckrichtung des aufzunehmenden Werkzeugs verengt.

Die Klemmarme 4 haben radial nach innen ragende Eingriffsabschnitte 10 zur kraft- und/oder formschlüssigen Kontaktierung des Werkzeugs, um das Werkzeug zu halten. Durch Einstecken des Werkzeugs (und das Kontaktieren der Eingriffsabschnitt 10) werden die Klemmarme 4 in Radialrichtung der Aufnahme-Expanderhülse 2 federelastisch nach außen gedrückt. Durch die dadurch resultierende elastische Spannung der Klemmarme 4 wird das eingesteckte Werkzeug zwischen den Klemmarmen 4 gehalten/festgeklemmt. Das heißt, dass ein Außendurchmesser eines radial innerhalb der Aufnahme-Expanderhülse 2 gebildeten Aufnahmeraums zur Aufnahme des Werkzeugs durch die Eingriffsabschnitte 10 bzw. der elastischen Verformbarkeit der Klemmarme 4 nach radial außen begrenzt ist. Hierbei sind die Eingriffsabschnitte 10 vorzugsweise so ausgebildet, dass die Kontaktfläche zwischen den Klemmarmen 4 und dem aufgenommenen Werkzeug möglichst gering (linien-und/oder punktförmig) ist. Da die Oberfläche des Werkzeugs nur an den durch die Eingriffsabschnitte 10 definierten Punkten/Linien eine Kontaktstelle mit der Aufnahme-Expanderhülse 2 bildet, ist das Werkzeug großflächig von Reinigungsfluid umspülbar.

Die Aufnahme-Expanderhülse 2 hat den Auffangboden 6, der an dem der Einstecköffnung 8 gegenüberliegenden Endabschnitt der Aufnahme-Expanderhülse 2 ausgebildet ist. Der Auffangboden 6 erstreckt sich im Wesentlichen plattenförmig senkrecht zur Axialrichtung der Aufnahme-Expanderhülse 2. Alternativ kann der Auffangboden auch mit einer Prägung ausgeführt sein. In der dargestellten Ausführungsform hat der Auffangboden 6 die Form eines Kreises. Alternativ könnte der Auffangboden 6 beispielsweise ringförmig, oval oder im Wesentlichen dreieckig ausgebildet sein. Der Auffangboden 6 könnte auch mit einem oder mehreren Löchern versehen sein oder mit einer zentralen, im Wesentlichen kegelstumpfförmigen Vertiefung, in die ein Ende eines Werkzeugs selbstzentrierend aufgenommen werden kann. Die Kegelstumpffläche dieser Vertiefung kann dabei auch geschlitzt bzw. unterbrochen ausgeführt sein und die "Spitze" des Kegelstumpfes kann offen oder geschlossen ausgebildet sein. Die Klemmarme 4 erstrecken sich von der radialen Außenkante des Auffangbodens 6 aus in Axialrichtung zu der offenen Stirnseite der Aufnahme-Expanderhülse 2 hin. Vorzugsweise ist der Auffangboden 6 aus einem Metall, beispielsweise aus einem Federstahl, wie dem Werkstoff 1.4310, gefertigt. Beispielsweise können die Klemmarme 4 über einen spitzwinkligen Eckverbinder/einen spitzwinkligen Eckverbindungsbereich/eine Biegestelle an dem Auffangboden 6 angebracht sein, so dass der Neigungswinkel an dem Eckverbinder zwischen den Klemmarmen 4 und dem Auffangboden 6 durch das Einstecken des Werkzeugs entgegen der Elastizität der Klemmarme 4 vergrößert wird. Insbesondere kann der Auffangboden 6 integral/werkstoffeinstückig mit den Klemmarmen 4 verbunden sein.

Die Aufnahme-Expanderhülse 2 kann in die Halterungsvorrichtung 50 des Werkzeugsets 100 eingesteckt werden. Insbesondere kann die Aufnahme-Expanderhülse 2 unmittelbar an der Halterungsvorrichtung 50 befestigt werden. Die Halterungsvorrichtung 50 weist eine erste Lochplatte (Lochmatrix/Hülsenblech/Siebstruktur) 52 und eine zweite Lochplatte (Gitterplatte/Lochmatrix/Bodenblech/Siebstruktur) 54 auf. Die beiden Lochplatten 52, 54 sind zueinander parallel beabstandet angeordnet und miteinander beispielsweise über Außenwände (einen Rahmen) 56 (vgl. Fig. 17 oder Fign. 27 und 28) oder Stege verbunden.

Die erste Lochplatte 52 hat eine Anzahl erste Löcher (Aussparungen/Perforationen) 58. Die ersten Löcher 58 sind in der dargestellten Ausführungsform im Wesentlichen rund ausgebildet und können zur Aufnahme unterschiedlicher Werkzeugschaftdurchmesser unterschiedliche Durchmesser haben. Alternativ können die ersten Löcher 58 auch eine im Wesentlichen rechteckige oder quadratische Form haben oder sternförmig sein, auch wenn dies nicht dargestellt ist. Die ersten Löcher 58 sind durch Material der ersten Lochplatte 52, beispielsweise durch erste Stege 60, voneinander getrennt. Beispielsweise können die ersten Löcher 58, wie in Fig. 1 dargestellt, in geraden Reihen angeordnet sein. Das heißt, dass die Lochmitten der ersten Löcher 58 benachbarter Reihen und/oder die Lochmitten von benachbarten ersten Löchern 58 einer Reihe jeweils den gleichen Abstand haben, d.h. die gleiche Lochteilung besitzen. In Fig. 1 ist die Aufnahme-Expanderhülse 2 in eines der ersten Löcher 58 in Einsteckrichtung des Werkzeugs eingesetzt, so dass es in Einsteckrichtung des Werkzeugs, d.h. in Richtung zu der zweiten Lochplatte 54 hin, in die erste Lochplatte 52 eintaucht.

Die zweite Lochplatte 54 hat eine Anzahl zweiter Löcher (Aussparungen/Perforationen) 62. Die zweiten Löcher 62 sind im Wesentlichen quadratisch (vgl. Fig. 2) oder im Wesentlichen rund ausgebildet (vgl. Fign. 10, 13 und 16). Die zweiten Löcher 62 sind durch Material der zweiten Lochplatte 54, beispielsweise durch zweite Stege 64, voneinander getrennt. Vorzugsweise bei quadratischer Form können die zweiten Löcher 62 beispielsweise in geraden Reihen angeordnet sein. Das heißt, dass die Lochmitten der zweiten Löcher 62 benachbarter Reihen und/oder die Lochmitten von benachbarten zweiten Löchern 62 einer Reihe jeweils den gleichen Abstand haben, d.h. die gleiche Lochteilung besitzen. Das heißt, dass die zweiten Stege 64 geradlinig sowie parallel bzw. senkrecht zueinander angeordnet sind. Die zweiten Stege 64 schneiden sich an ihren Kreuzungspunkten 66.

Die ersten Löcher 58 und die zweiten Löcher 62 können in Axialrichtung zueinander fluchtend angeordnet sein. Alternativ können die zweiten Löcher 62 auch in Axialrichtung nicht fluchtend zu den ersten Löchern 58 angeordnet sein, wobei vorzugsweise bei nicht fluchtender Anordnung die Kreuzungspunkte 66 in Axialrichtung fluchtend zu den ersten Löchern 58 angeordnet sind.

Zumindest eines der ersten Löcher 58 kann in einer vorteilhaften Weiterbildung (vgl. Fign. 3 und 4) radial nach innen ragende, durch Material der ersten Lochplatte 52 gebildete Trennstege 68 aufweisen. Die Trennstege 68 dienen als formschlüssige Verdrehsicherung für die Aufnahme-Expanderhülse 2. Vorzugsweise weist das zumindest eine Loch der ersten Löcher 58 (je später detailliert beschriebenem Befestigungsabschnitt 14) zumindest zwei radial nach innen ragende Trennstege 68 auf, die jeweils einen Anschlag in einer Umfangsrichtung der Aufnahme-Expanderhülse 2 bilden. Das heißt, dass jeder der Befestigungsabschnitte 14 in Umfangsrichtung der Aufnahme-Expanderhülse 2 zwischen zwei Trennstegen 68 angeordnet ist. Durch die Trennstege 68 wird das zumindest eine Loch der ersten Löcher 58 in mehrere voneinander getrennte Lochabschnitte unterteilt. Vorzugsweise können die Trennstege 68 derart ausgebildet sein, insbesondere (etwa jeweils zwei Trennstege 68) derart miteinander verbunden sein, dass sie, insbesondere zusammen mit dem Außendurchmesser des Lochs, mehrere im Wesentlichen dreieckige oder kreissektorförmige Befestigungsabschnittsperforationen 70 bilden, durch die jeweils ein Befestigungsabschnitt 14 (axial, vorzugsweise entgegen der Einsteckrichtung) durchführbar ist. Zudem oder alternativ können die Trennstege 68 derart ausgebildet sein, insbesondere (etwa jeweils zwei Trennstege 68) derart miteinander verbunden sein, dass sie, insbesondere zusammen mit dem Außendurchmesser des Lochs, eine sternförmige Klemmarmperforation 72 bilden. Die Klemmarmperforation 72 hat vorzugsweise sich von der Lochmitte sternförmig radial nach außen erstreckende Ausweichspalte, deren Breite größer als eine Breite der Klemmarme 4 ist. Dadurch können die Klemmarme 4 innerhalb der Klemmarmperforation 72, vorzugsweise durch die Trennstege 68 geführt, elastisch in Radialrichtung verlagert werden. Vorzugsweise hat die Klemmarmperforation 72 zumindest so viele Ausweichspalte wie die Aufnahme-Expanderhülse 2 Klemmarme 4 hat.

Die zweiten Löcher 62 können in einer vorteilhaften Weiterbildung (vgl. Fign. 10 und 11 oder Fign. 13 und 14) radial nach innen ragende, durch Material der zweiten Lochplatte 54 gebildete Trennstege 74 aufweisen. Vorzugsweise weist zumindest ein Loch der zweiten Löcher 62 die radial nach innen ragenden Trennstege 74 auf, die das unterteilte zweite Loch 62 in mehrere Lochabschnitte 76 unterteilen. Die Trennstege 74 können sich vorzugsweise in einem Mittelpunkt des unterteilten zweiten Lochs 62 schneiden. Die Lochabschnitte 76 weisen vorzugsweise die gleiche Form auf und können beispielsweise im Wesentlichen dreieckig oder kreissektorförmig ausgebildet sein. Das heißt, dass die Trennstege 74 derart ausgebildet sein können, insbesondere derart miteinander verbunden sein können, dass sie sich an der Lochmitte überschneiden und sternförmig von der Lochmitte radial nach außen erstrecken und dadurch, insbesondere zusammen mit dem Außendurchmesser des Lochs, die mehreren im Wesentlichen dreieckigen oder kreissektorförmigen Lochabschnitte 76 bilden.

Die Aufnahme-Expanderhülse 2 hat mehrere, beispielsweise an Befestigungsarmen 12 (vgl. Fign. 1 bis 15) ausgebildete Befestigungsabschnitte 14, die zur kraft- und formschlüssigen Befestigung der Aufnahme-Expanderhülse 2 an der Halterungsvorrichtung 50 des Werkzeugsets dienen. In der dargestellten Ausführungsform hat die Aufnahme-Expanderhülse 2 drei umfangsbeabstandete Befestigungsarme 12. Alternativ könnte die Aufnahme-Expanderhülse 2 auch mehr als drei Befestigungsarme 12 haben. Die Befestigungsarme 12 können gleichverteilt über den Umfang der Aufnahme-Expanderhülse 2 angeordnet sein. Vorzugsweise entspricht die Anzahl der Befestigungsarme 12 der Anzahl der Klemmarme 4. Insbesondere können die Befestigungsarme 12 in Umfangsrichtung der Aufnahme-Expanderhülse 2 abwechselnd mit den Klemmarmen 4 angeordnet sein. Vorzugsweise sind die Befestigungsarme 12 aus einem Metall, beispielsweise aus einem Federstahl, wie dem Werkstoff 1.4310, gefertigt. Beispielsweise können die die Befestigungsarme 12 fest, insbesondere über einen Eckverbinder/einen Eckverbindungsbereich/eine Biegestelle an dem Auffangboden 6 angebracht sein. Insbesondere können die Befestigungsarme 12 integral/werkstoffeinstückig mit dem Auffangboden 6 und/oder mit den Klemmarmen 4 verbunden sein.

Die Befestigungsabschnitte 14 dienen insbesondere zur unmittelbaren form- und kraftschlüssigen Befestigung an der Halterungsvorrichtung 50 des Werkzeugsets 100, insbesondere an der ersten Lochplatte 52 und/oder der zweiten Lochplatte 54. Die Befestigungsabschnitte 14 sind angepasst und vorgesehen, um unmittelbar mit der ersten Lochplatte 52 oder zweiten Lochplatte 54 zum Befestigen an dieser Lochplatte 52 zusammenzuwirken und diese Lochplatte 52, 54 radial klemmend derart axial zu umgreifen, dass sie das Lochplattenmaterial in Lochplattendickenrichtung formschlüssig entgegen einer Einsteckrichtung des einzusteckenden Werkzeugs hintergreifen.

Der Auffangboden 6 der Aufnahme-Expanderhülse 2 ist durch eine ebene Platte gebildet. Alternativ kann der Auffangboden 6 auch gewölbt sein, beispielsweise im Wesentlichen kegelstumpfförmig ausgebildet sein, oder eine Prägung, einen Überstand oder dergleichen aufweisen, auch wenn dies nicht dargestellt ist. Eine Unterseite 16 des Auffangbodens 6 ist eine von den Klemmarmen 4 abgewandte Axialseite des Auffangsbodens 6, d.h. eine axiale Außenfläche der Aufnahme-Expanderhülse 2. Eine Oberseite 18 des Auffangbodens 6 ist eine den Klemmarmen 4 zugewandte Axialseite des Auffangsbodens 6, d.h. eine (zwischen den Klemmarmen 4 angeordnete) axiale Innenfläche der Aufnahme-Expanderhülse 2. Die Oberseite 18 des Auffangbodens 6 bildete eine axiale Anschlagsfläche für das einzusteckende Werkzeug.

In der in Fign. 1 bis 9 dargestellten ersten Modifikation erstrecken sich die Befestigungsarme 12 von dem Auffangboden 6 aus in Axialrichtung und bilden an ihren freien, radial nach außen gebogenen Endabschnitten die Befestigungsabschnitte 14, etwa in Form von nach radial außen ausgerichteten Rastnasen, aus. Dabei erstrecken sich die Befestigungsarme 12 vom Auffangboden 6 aus in die gleiche Richtung wie die Klemmarme 4. Die in Fig. 1 bis 9 dargestellten Befestigungsarme 12 erstrecken sich im Wesentlichen genauso weit in Axialrichtung wie die Klemmarme 4. Insbesondere entspricht eine Axialerstreckung der in Fig. 1 bis 9 dargestellten Befestigungsarme 12 im Wesentlichen einem Abstand zwischen der ersten Lochplatte 52 und der zweiten Lochplatte 54. Die radial nach außen gebogenen oder abstehenden Endabschnitte, welche die Befestigungsabschnitte 14 ausbilden, sind dazu angepasst und vorgesehen, eines der ersten Löcher 58 zu durchgreifen, so dass sie von radial innen klemmend das Lochplattenmaterial der ersten Lochplatte 52 in Lochplattendickenrichtung hintergreifen. Mit anderen Worten umklammern die Befestigungsabschnitte 14 die erste Lochplatte 52 von unten.

In der in Fign. 10 bis 12 dargestellten zweiten Modifikation erstrecken sich die Befestigungsarme 12 von dem Auffangboden 6 aus in Axialrichtung erstrecken und bilden an ihren freien, radial nach innen gebogenen Endabschnitten die Befestigungsabschnitte 14, etwa in Form von Befestigungsklauen, aus. Dabei erstrecken sich die Befestigungsarme 12 vom Auffangboden 6 aus in eine entgegengesetzte Richtung gegenläufig zu den Klemmarmen 4. Die in Fig. 10 bis 12 dargestellten Befestigungsarme 12 sind in Axialrichtung (wesentlich) kürzer als die Klemmarme 4 ausgebildet. Insbesondere entspricht eine Axialerstreckung der in Fig. 10 bis 12 dargestellten Befestigungsarme 12 im Wesentlichen der Dicke der zweiten Lochplatte 54. Die radial nach innen gebogenen oder abstehenden Endabschnitte, welche die Befestigungsabschnitte 14 ausbilden, sind dazu angepasst und vorgesehen, eines der zweiten Löcher 62 (bzw. deren Lochabschnitte 74) zu durchgreifen, so dass sie von radial außen klemmend das Lochplattenmaterial der zweiten Lochplatte 54 in Lochplattendickenrichtung hintergreifen. Der Auffangboden 6 liegt mit seiner Unterseite 16 axial auf Lochplattenmaterial der zweiten Lochplatte 54 auf. Mit anderen Worten umklammern die Befestigungsabschnitte 14 die zweite Lochplatte 54 von oben.

In der in Fign. 13 bis 15 dargestellten dritten Modifikation erstrecken sich die Befestigungsarme 12 von dem Auffangboden 6 aus in Axialrichtung erstrecken und bilden an ihren freien, radial nach innen gebogenen Endabschnitten die Befestigungsabschnitte 14, etwa in Form von Befestigungsklauen, aus. Dabei erstrecken sich die Befestigungsarme 12 vom Auffangboden 6 aus in die gleiche Richtung wie die Klemmarme 4. Die in Fig. 13 bis 15 dargestellten Befestigungsarme 12 sind in Axialrichtung (wesentlich) kürzer als die Klemmarme 4 ausgebildet. Insbesondere entspricht eine Axialerstreckung der in Fig. 13 bis 15 dargestellten Befestigungsarme 12 im Wesentlichen der Dicke der zweiten Lochplatte 54. Die radial nach innen gebogenen oder abstehenden Endabschnitte, welche die Befestigungsabschnitte 14 ausbilden, sind dazu angepasst und vorgesehen, eines der zweiten Löcher 62 (bzw. deren Lochabschnitte 74) zu durchgreifen, so dass sie von radial außen klemmend das Lochplattenmaterial der zweiten Lochplatte 54 in Lochplattendickenrichtung hintergreifen. Der Auffangboden 6 liegt mit seiner Oberseite 16 axial an dem Lochplattenmaterial der zweiten Lochplatte 54 an. Mit anderen Worten umklammern die Befestigungsabschnitte 14 die zweite Lochplatte 54 von unten.

In der in Fign. 16 bis 21 dargestellten vierten Modifikation bilden die Klemmarme 4 an einem ersten Axialabschnitt die Eingriffsabschnitte 10 aus und an einem sich in Axialrichtung unmittelbar daran anschließenden zweiten Axialabschnitt die Befestigungsabschnitte 14, etwa in Form von Krümmungsabschnitten, aus. Der zweite Axialabschnitt befindet sich gegenüber dem ersten Axialabschnitt radial außerhalb. Der zweite Axialabschnitt ist zwischen dem Auffangboden und dem ersten Axialabschnitt des Klemmarms 4 ausgebildet. Der zweite Axialabschnitt ist ein radial nach innen gebogener, etwa U-förmiger Abschnitt, des Klemmarms 4 und dazu angepasst und vorgesehen, eines der zweiten Löcher 62 der zweiten Lochplatte 54 zu durchgreifen, so dass er von radial innen klemmend das Lochplattenmaterial in Lochplattendickenrichtung hintergreift bzw. umgreift. Der U-förmige Abschnitt kann vorzugsweise eine nach radial außen ausgerichtete Öffnung besitzen. Im montierten Zustand befindet sich der Auffangboden 6 der Aufnahme-Expanderhülse 2 vorzugsweise unterhalb der zweiten Lochplatte 54 (d.h. außerhalb des Zwischenraums zwischen der ersten Lochplatte 52 und der zweiten Lochplatte 54).

In der in Fign. 22 und 23 dargestellten fünften Modifikation bilden die Klemmarme 4 an einem ersten Axialabschnitt die Eingriffsabschnitte 10 aus und an einem sich in Axialrichtung unmittelbar daran anschließenden zweiten Axialabschnitt die Befestigungsabschnitte 14 aus. Der zweite Axialabschnitt befindet sich gegenüber dem ersten Axialabschnitt radial außerhalb. Der zweite Axialabschnitt ist an einem freien Endabschnitt des Klemmarms 4 ausgebildet. Der zweite Axialabschnitt ist ein radial nach innen gebogener, etwa U-förmigen Abschnitt, des Klemmarms 4 ist und dazu angepasst und vorgesehen, eines der ersten Löcher 58 der ersten Lochplatte 52 zu durchgreifen, so dass er von radial innen klemmend das Lochplattenmaterial in Lochplattendickenrichtung hintergreift bzw. umgreift. Der U-förmige Abschnitt kann vorzugsweise eine nach radial außen ausgerichtete Öffnung besitzen. Die in Fign. 22 bis 23 dargestellte fünfte Modifikation der Aufnahme-Expanderhülse 2 kann an einer als Einzelblech/Einzellochplatte ausgebildeten Halterungsvorrichtung 50 befestigt werden.

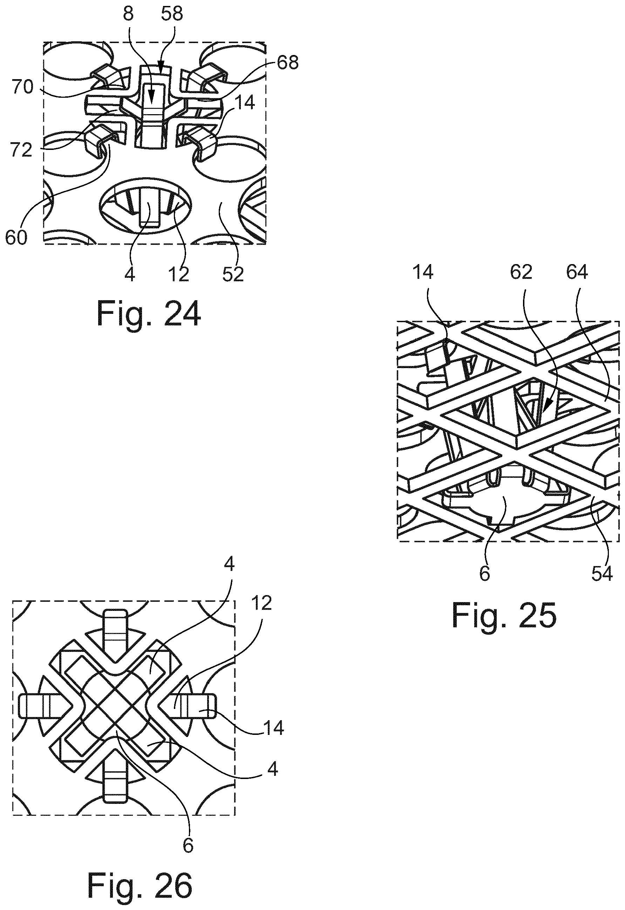

Fign. 24 bis 26 zeigen eine zweite Ausführungsform der vorliegenden Offenbarung. Die in die Haltevorrichtung 50 eingehängte Aufnahme-Expanderhülse 2 der zweiten Ausführungsform ist in Fig. 24 in einer perspektivischen Ansicht von oben, in Fig. 25 in einer perspektivischen Ansicht von unten und in Fig. 26 in einer Draufsicht dargestellt.

Die Aufnahme-Expanderhülse 2 hat mehrere, in der dargestellten Ausführungsform vier, umfangsbeabstandete Klemmarme 4. Alternativ könnte die Aufnahme-Expanderhülse 2 beispielsweise auch drei Klemmarme 4 oder mehr als vier Klemmarme 4 haben. Die Klemmarme 4 können gleichverteilt über den Umfang der Aufnahme-Expanderhülse 2 angeordnet sein. Die Klemmarme 4 sind elastisch verformbar und bilden an der offenen Stirnseite der Aufnahme-Expanderhülse 2 die Einstecköffnung 8 für das Einstecken des (nicht dargestellten) Werkzeugs aus. Vorzugsweise sind die Klemmarme 4 biegeelastisch und aus einem Metall, beispielsweise aus einem Federstahl, wie dem Werkstoff 1.4310, gefertigt. Die Klemmarme 4 bilden an ihren (stirnseitigen) freien Endabschnitten vorzugsweise einen Trichter, der sich in Einsteckrichtung des aufzunehmenden Werkzeugs verengt.

Die Klemmarme 4 haben die radial nach innen ragende Eingriffsabschnitte 10 zur kraft- und/oder formschlüssigen Kontaktierung des Werkzeugs, um das Werkzeug zu halten. Durch Einstecken des Werkzeugs (und das Kontaktieren des Eingriffsabschnitts 10) werden die Klemmarme 4 in Radialrichtung federelastisch nach außen gedrückt. Durch die dadurch resultierende elastische Spannung der Klemmarme 4 wird das eingesteckte Werkzeug zwischen den Klemmarmen 4 gehalten/festgeklemmt. Das heißt, dass ein Außendurchmesser eines radial innerhalb der Aufnahme-Expanderhülse 2 gebildeten Aufnahmeraums zur Aufnahme des Werkzeugs durch die Eingriffsabschnitte 10 bzw. der elastischen Verformbarkeit der Klemmarme 4 nach radial außen begrenzt ist. Hierbei sind die Eingriffsabschnitte 10 vorzugsweise so ausgebildet, dass die Kontaktfläche zwischen den Klemmarmen 4 und dem aufgenommenen Werkzeug möglichst gering (linien- und/oder punktförmig) ist. Da die Oberfläche des Werkzeugs nur an den durch die Eingriffsabschnitte 10 definierten Punkten/Linien eine Kontaktstelle mit der Aufnahme-Expanderhülse 2 bildet, ist das Werkzeug großflächig von Reinigungsfluid umspülbar.

Die Aufnahme-Expanderhülse 2 hat den Auffangboden 6, der an dem der Einstecköffnung 8 gegenüberliegenden Endabschnitt der Aufnahme-Expanderhülse 2 ausgebildet ist. Der Auffangboden 6 kann als Axialanschlag dienen und ein zu tiefes Einstecken des Werkzeugs verhindern. Der Auffangboden 6 erstreckt sich im Wesentlichen plattenförmig senkrecht zur Axialrichtung der Aufnahme-Expanderhülse 2. In der dargestellten Ausführungsform hat der Auffangboden 6 die Form eines Kreises. Alternativ könnte der Auffangboden 6 beispielsweise ringförmig, oval oder im Wesentlichen dreieckig ausgebildet sein. Der Auffangboden 6 kann eben oder gewölbt, beispielsweise im Wesentlichen kegelstumpfförmig, ausgebildet sein. Von dem Auffangboden 6 aus erstrecken sich die Klemmarme 4 in Axialrichtung zu der Einstecköffnung 8 hin. Die Klemmarme 4 erstrecken sich von der radialen Außenkante des Auffangbodens 6 aus. Vorzugsweise ist der Auffangboden 6 aus einem Metall, beispielsweise aus einem Federstahl, wie dem Werkstoff 1.4310, gefertigt. Beispielsweise können die Klemmarme 4 über einen spitzwinkligen Eckverbinder/einen spitzwinkligen Eckverbindungsbereich/eine Biegestelle an dem Auffangboden 6 angebracht sein, so dass der Neigungswinkel an dem Eckverbinder zwischen den Klemmarmen 4 und dem Auffangboden 6 durch das Einstecken des Werkzeugs entgegen der Elastizität der Klemmarme 4 vergrößert wird. Insbesondere kann der Auffangboden 6 integral/werkstoffeinstückig mit den Klemmarmen 4 verbunden sein.

Die Aufnahme-Expanderhülse 2 hat mehrere Befestigungsarme 12, die zur Befestigung der Aufnahme-Expanderhülse 2 an der Halterungsvorrichtung 50 des Werkzeugsets dienen. In der dargestellten Ausführungsform hat die Aufnahme-Expanderhülse 2 vier umfangsbeabstandete Befestigungsarme 12. Alternativ könnte die Aufnahme-Expanderhülse 2 beispielsweise auch drei Befestigungsarme 12 oder mehr als vier Befestigungsarme 12 haben. Die Befestigungsarme 12 können gleichverteilt über den Umfang der Aufnahme-Expanderhülse 2 angeordnet sein. Vorzugsweise entspricht die Anzahl der Befestigungsarme 12 der Anzahl der Klemmarme 4. Insbesondere können die Befestigungsarme 12 in Umfangsrichtung abwechselnd mit den Klemmarmen 4 angeordnet sein. Vorzugsweise sind die Befestigungsarme 12 aus einem Metall, beispielsweise aus einem Federstahl, wie dem Werkstoff 1.4310, gefertigt. Beispielsweise können die die Befestigungsarme 12 fest, insbesondere über einen Eckverbinder/einen Eckverbindungsbereich/eine Biegestelle an dem Auffangboden 6 angebracht sein. Insbesondere können die Befestigungsarme 12 integral/werkstoffeinstückig mit dem Auffangboden 6 und/oder mit den Klemmarmen 4 verbunden sein. Die Befestigungsarme 12 erstrecken sich von dem Auffangboden 6 aus in Axialrichtung zu der Einstecköffnung 8 hin. Die Befestigungsarme 12 erstrecken sich von der radialen Außenkante des Auffangbodens 6 aus. Vorzugsweise ist die Aufnahme-Expanderhülse 2 als ein metallisches Biegeumformteil ausgebildet.

Die Befestigungsarme 12 haben an ihren an der offenen Stirnseite der Aufnahme-Expanderhülse 2 angeordneten Endabschnitten jeweils einen hakenartigen Befestigungsabschnitt 14 zur einhängenden Befestigung an der Halterungsvorrichtung 50. Der Befestigungsabschnitt 14 ist derart gebogen oder abgewinkelt ausgebildet, dass er (in Einsteckrichtung des Werkzeugs) an der Halterungsvorrichtung 50 einhängbar ist. Vorzugsweise kann der Befestigungsabschnitt 14 derart ausgebildet und vorgesehen sein, dass er die Halterungsvorrichtung 50 radial und axial hintergreift. Mit anderen Worten umgreift der Befestigungsabschnitt 14 die Halterungsvorrichtung 50 axial und radial, insbesondere zur Festlegung der Radialposition und der Axialposition der Aufnahme-Expanderhülse 2 an der Halterungsvorrichtung 50.

Insbesondere kann der Befestigungsabschnitt 14 ein U-förmiges Profil bilden. In der dargestellten Ausführungsform erstreckt sich ein Basisabschnitt, der einen Grund des U-förmigen Profils bildet, im Wesentlichen in Radialrichtung (d.h. senkrecht zu der Axialrichtung). Der Basisabschnitt liegt also flächig an der Halterungsvorrichtung 50 an. Schenkel des U-förmigen Profils erstrecken sich von den Enden des Basisabschnitts aus im Wesentlichen in Axialrichtung in die gleiche Richtung. Ein radial innerer Schenkel der beiden Schenkel kann sich in Axialrichtung länger, vorzugsweise bis zu dem Auffangboden 6 hin, erstrecken als ein radial äußerer Schenkel der beiden Schenkel. Das heißt, dass das Profil der Befestigungsabschnitte 14 eine Einstecköffnung (Einhängeöffnung) hat, die vorzugsweise radial nach außen und/oder vorzugsweise axial zu dem Auffangboden 6 hin, d.h. entgegen der Einsteckrichtung des Werkzeugs, ausgerichtet sein kann. Durch die radial nach außen ausgerichtete Einstecköffnung des U-Profils können die Befestigungsabschnitte 14 von radial innen an der Halterungsvorrichtung 50 angebracht werden. Durch die axial entgegen der Einsteckrichtung des Werkzeugs ausgerichtete Einstecköffnung des U-Profils können die Befestigungsabschnitte 14 in der Einsteckrichtung des Werkzeugs an der Halterungsvorrichtung 50 angebracht werden.

Der Auffangboden 6 der Aufnahme-Expanderhülse 2 ist beispielsweise durch eine ebene Platte gebildet. Alternativ kann der Auffangboden 6 auch gewölbt sein, beispielsweise im Wesentlichen kegelstumpfförmig sein, oder eine Prägung, einen Überstand oder dergleichen aufweisen, auch wenn dies nicht dargestellt ist. Die Unterseite 16 des Auffangbodens 6 ist die von den Klemmarmen 4 abgewandte Axialseite des Auffangsbodens 6, d.h. eine axiale Außenfläche der Aufnahme-Expanderhülse 2. Die Oberseite 18 des Auffangbodens 6 ist die den Klemmarmen 4 zugewandte Axialseite des Auffangsbodens 6, d.h. eine (zwischen den Klemmarmen 4 angeordnete) axiale Innenfläche der Aufnahme-Expanderhülse 2. Die Oberseite 18 des Auffangbodens 6 bildet eine axiale Anschlagsfläche für das einzusteckende Werkzeug.

Die Halterungsvorrichtung 50 der zweiten Ausführungsform weist die erste Lochplatte 52 auf. Die erste Lochplatte 52 hat eine Anzahl erste Löcher (Aussparungen/Perforationen) 58, die in der dargestellten Ausführungsform rund ausgebildet sind und zur Aufnahme unterschiedlicher Werkzeugschaftdurchmesser unterschiedliche Durchmesser haben. Alternativ können die ersten Löcher 58 auch eine im Wesentlichen rechteckige oder quadratische Form haben oder sternförmig sein, auch wenn dies nicht dargestellt ist. Die ersten Löcher 58 sind durch erste Stege 60 voneinander getrennt. Beispielsweise können die ersten Löcher 58, wie in Fig. 24 dargestellt, in 60° versetzten Reihen angeordnet sein. Das heißt, dass die Lochmitten benachbarter Reihen mittig zueinander versetzt angeordnet sind. Die Halterungsvorrichtung 50 kann die zweite Lochplatte 54 aufweisen, die parallel zu der ersten Lochplatte 52 beabstandet angeordnet ist und mit der ersten Lochplatte 52, beispielsweise über den in Fign. 24 bis 26 nicht dargestellten Rahmen, verbunden ist. Die zweite Lochplatte 54 hat eine Anzahl zweite Löcher (Aussparungen/Perforationen) 62, die eine quadratische Form haben können. Beispielsweise können die Löcher 62, wie in Fig. 25 dargestellt, in geraden Reihen angeordnet sein. Die Löcher 62 können von geradlinigen Stegen 64 voneinander getrennt sein. Die zweite Lochplatte 54 kann als Verdrehsicherung für die Aufnahme-Expanderhülse 2 dienen. Die in Fign. 24 bis 26 dargestellte zweite Ausführungsform der Aufnahme-Expanderhülse 2 kann auch an einer als Einzelblech/Einzellochplatte ausgebildeten/nur die erste Lochplatte 52 aufweisenden Halterungsvorrichtung 50 befestigt werden.

Die Aufnahme-Expanderhülse 2 der zweiten Ausführungsform ist an der ersten Lochplatte 52 der Halterungsvorrichtung 50 befestigt. Insbesondere ist die Aufnahme-Expanderhülse 2 in eines der ersten Löcher 58 eingesteckt. Der hakenartige Befestigungsabschnitt 14 hintergreift den Steg 60 axial und radial, so dass er, insbesondere der radial äußere Schenkel des U-förmigen Profils, in ein benachbartes erstes Loch 58 formschlüssig eingreift. Mit anderen Worten durchgreift der Befestigungsabschnitt 14 das erste Loch 58, in das die Aufnahme-Expanderhülse 2 eingesteckt ist, radial nach außen abgewinkelt, so dass er sich an dem Lochplattenmaterial parallel/entlang nach außen erstreckt und den das erste Loch 58 begrenzenden Steg 60 axial hintergreift, und ist axial in Richtung zu dem Auffangboden 6 hin abgewinkelt, so dass er in das benachbarte erste Loch 58 formschlüssig eingreift und den das erste Loch 58 begrenzenden Steg 60 radial hintergreift. Somit umgreift der Befestigungsabschnitt 14 das Lochplattenmaterial in Axialrichtung, insbesondere entgegen der Einsteckrichtung des Werkzeugs. Das heißt, dass die Befestigungsabschnitte 14 an den Stegen 60 der ersten Lochplatte 52 eingehängt sind.

Zumindest eines der ersten Löcher 58 kann in einer vorteilhaften Weiterbildung die radial nach innen ragenden, durch Material der ersten Lochplatte 52 gebildeten Trennstege 68 aufweisen. Die Trennstege 68 bilden eine formschlüssige Verdrehsicherung für die Aufnahme-Expanderhülse 2, insbesondere für die Befestigungsabschnitte 14. Vorzugsweise weist das zumindest eine Loch der ersten Löcher 58 (je Befestigungsabschnitt 14) zumindest zwei radial nach innen ragende Trennstege 68 auf, die jeweils einen Anschlag in einer Umfangsrichtung der Aufnahme-Expanderhülse 2 bilden. Das heißt, dass jeder der Befestigungsabschnitte 14 in Umfangsrichtung der Aufnahme-Expanderhülse 2 zwischen zwei Trennstegen 68 angeordnet ist.

Durch die Trennstege 68 wird das zumindest eine Loch der ersten Löcher 58 in mehrere voneinander getrennte Lochabschnitte unterteilt. Vorzugsweise können die Trennstege 68 derart ausgebildet sind, insbesondere (etwa jeweils zwei Trennstege 68) derart miteinander verbunden sein, dass sie, insbesondere zusammen mit dem Außendurchmesser des Lochs, mehrere im Wesentlichen dreieckige oder kreissektorförmige Befestigungsabschnittsperforationen 70 bilden. Jede Befestigungsabschnittsperforation 70 ist auf den Befestigungsabschnitt 14, insbesondere der Basisabschnitt des U-Profils, derart abgestimmt, dass der Befestigungsabschnitt 14 durch die Befestigungsabschnittsperforation 70 axial (vorzugsweise entgegen der Einsteckrichtung) durchführbar ist. Zudem oder alternativ können die Trennstege 68 derart ausgebildet sein, insbesondere (etwa jeweils zwei Trennstege 68) derart miteinander verbunden sein, dass sie, insbesondere zusammen mit dem Außendurchmesser des Lochs, eine sternförmige Klemmarmperforation 72 bilden. Die Klemmarmperforation 72 hat vorzugsweise sich von der Lochmitte sternförmig radial nach außen erstreckende Ausweichspalte, deren Breite größer als eine Breite der Klemmarme 4 ist. Dadurch können die Klemmarme 4 innerhalb der Klemmarmperforation 72, vorzugsweise durch die Trennstege 68 geführt, elastisch in Radialrichtung (begrenzt) verlagert werden. Vorzugsweise hat die Klemmarmperforation 72 zumindest so viele Ausweichspalte wie die Aufnahme-Expanderhülse 2 Klemmarme 4 hat. In der in Fign. 24 bis 26 dargestellten Ausführungsform mit vier Klemmarmen 4 hat die Klemmarmperforation 72 einen plusförmigen/kreuzförmigen Querschnitt. Ein durch die Trennstege 68 gebildeter Innendurchmesser kann den Schaftdurchmesser des einzusteckenden Werkzeugs begrenzen.

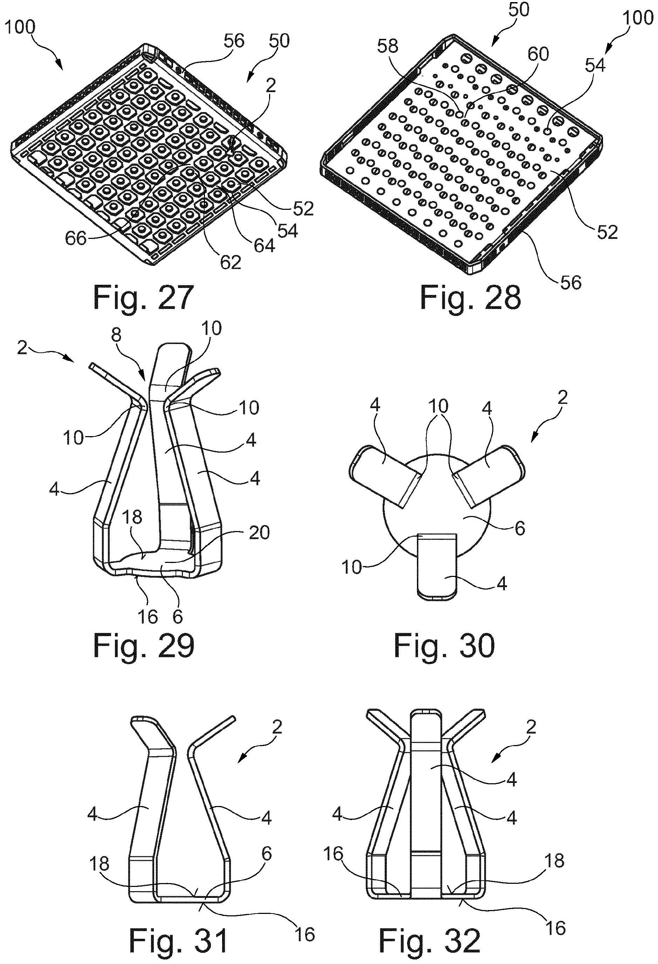

In Fign. 27 bis 38 ist eine dritte Ausführungsform dargestellt. Die Halterungsvorrichtung 50 der dritten Ausführungsform ist in Fig. 27 perspektivisch von unten und in Fig. 28 perspektivisch von oben dargestellt. Die erste Lochplatte (Lochmatrix/Hülsenblech/Siebstruktur) 52 und die zweite Lochplatte (Gitterplatte/Lochmatrix/Bodenblech/Siebstruktur) 54 sind zueinander parallel beabstandet angeordnet und miteinander über die Außenwände (Rahmen) 56 verbunden.

Die erste Lochplatte 52 hat eine Anzahl erster Löcher (Aussparungen/Perforationen) 58, die in der dargestellten Ausführungsform rund ausgebildet sind und zur Aufnahme unterschiedlicher Werkzeugschaftdurchmesser unterschiedliche Durchmesser haben. Alternativ können die ersten Löcher 58 auch eine im Wesentlichen rechteckige oder quadratische Form haben oder sternförmig sein, auch wenn dies nicht dargestellt ist. Alternativ könnten die ersten Löcher 58 mit gleichem Durchmesser ausgebildet sein. In jedes der ersten Löcher 58 ist jeweils eine Aufnahme-Expanderhülse 2 einsteckbar/einsetzbar bzw. eingesteckt/eingesetzt. Die ersten Löcher 58 sind durch erste Stege 60 voneinander getrennt.

Die zweite Lochplatte 54 hat eine Anzahl zweiter Löcher (Aussparungen/Perforationen) 62, die im Wesentlichen viereckig, vorzugsweise im Wesentlichen rechteckig oder quadratisch, ausgebildet sind und voneinander durch die zweiten Stege 64 getrennt werden. In der dargestellten Ausführungsform hat jedes der zweite Löcher 62 hat dieselbe Form. Alternativ könnten die zweiten Löcher 62 unterschiedliche Größen aufweisen.

Die zweiten Stege 62 erstrecken sich geradlinig und parallel zu den Außenkanten der zweiten Lochplatte 54. Die zweiten Stege 62 bilden eine Gitterstruktur und schneiden sich im Wesentlichen rechtwinklig an ihren Kreuzungspunkten 66. Die zweiten Löcher 62 sind in Reihen angeordnet und die zweiten Löcher 62 direkt benachbarter Reihen sind zueinander, vorzugsweise mittig, versetzt angeordnet. Somit sind die zweiten Löcher 62 in 60°-versetzten Reihen angeordnet. Das heißt, dass die zweiten Stege 64 an ihren Kreuzungspunkten 66 eine T-Kreuzung bilden bzw. dass jeder Kreuzungspunkt 66 den Rand von drei aneinander angrenzenden zweiten Löchern 62, insbesondere zwei Eckpunkte und eine Seitenkante, bildet. An den Kreuzungspunkten 66 verbreitern sich die zweiten Stege 64 an den Eckpunkten der zweiten Löcher 62 konvex nach außen, d.h. dass sie sich konvex in Richtung zur Lochmitte hin wölben. Dadurch ergibt sich, dass die zweiten Löcher 62 eine im Wesentlichen quadratische Form haben, deren Ecken konkav nach innen gewölbt sind (deren Eckpunkte sich etwa viertelkreisförmig nach innen wölben). Anders gesagt sind die zweiten Stege 64 derart verbreitert ausgebildet, dass an ihren Kreuzungspunkten 66 eine Kreisfläche, deren Radius im Wesentlichen der Breite der zweiten Stege 64 entspricht, vollständig zwischen den zweiten Löchern 62 angeordnet werden kann. Mit anderen Worten bilden die zweiten Stege 64 an den Kreuzungspunkten 66 eine Schweiß-, Löt- oder Klebfläche, die vorzugsweise im Wesentlichen so groß wie eine Fläche des Auffangbodens 6 ist.

Die ersten Löcher 58 sind zu den Kreuzungspunkten 66 der zweiten Lochplatte 54, insbesondere jeweils zu dem Mittelpunkt der zwischen den zweiten Löchern 62 ausgebildeten Kreisflächen, fluchtend angeordnet. Das heißt, dass die ersten Löcher 58 zu den zweiten Löchern 60 versetzt (d.h. nicht fluchtend) angeordnet sind. Insbesondere können die ersten Löcher 58 der ersten Lochplatte 52 in parallel beabstandeten Reihen angeordnet sein und die Lochmitten jeweils zwei benachbarter Reihen um eine Stegbreite der zweiten Stege 64 zueinander versetzt angeordnet sein.

Fign. 29 bis 32 zeigen verschiedene Ansichten der Aufnahme-Expanderhülse 2 der dritten Ausführungsform. Die Aufnahme-Expanderhülse 2 weist die elastisch verformbaren Klemmarme 4 auf. Die Klemmarme 4 bilden an ihren (stirnseitigen) freien Endabschnitten vorzugsweise einen Trichter, der sich in Einsteckrichtung des aufzunehmenden Werkzeugs verengt. Durch Einstecken des Werkzeugs wird der Durchmesser des durch die Klemmarme 4 radial begrenzten Hohlraums radial aufgeweitet und bilden Anlaufschrägflächen für das einzusteckende Werkzeug und die Klemmarme 4 werden in Radialrichtung federelastisch nach außen gedrückt. Durch die dadurch resultierende elastische Spannung der Klemmarme 4 wird das eingesteckte Werkzeug in dem Hohlraum zwischen den Klemmarmen 4 festgeklemmt. Hierbei sind die Klemmarme 4 vorzugsweise so ausgebildet, dass die Kontaktfläche zwischen den Klemmarmen und dem aufgenommenen Werkzeug möglichst gering (linien- und/oder punktförmig) ist. Da die Oberfläche des Werkzeugs nur an den durch die Eingriffsabschnitte 10 definierten Punkten/Linien eine Kontaktstelle mit der Aufnahme-Expanderhülse 2 bildet, ist das Werkzeug großflächig von Reinigungsfluid umspülbar.

Die Aufnahme-Expanderhülse 2 hat den Auffangboden 6, der an dem der Einstecköffnung 8 gegenüberliegenden Endabschnitt der Aufnahme-Expanderhülse 2 ausgebildet ist. Der Auffangboden 6 kann als Axialanschlag dienen und ein zu tiefes Einstecken des Werkzeugs verhindern. Der Auffangboden 6 erstreckt sich im Wesentlichen plattenförmig senkrecht zur Axialrichtung der Aufnahme-Expanderhülse 2. In der dargestellten Ausführungsform hat der Auffangboden 6 die Form eines Kreises. Alternativ könnte der Auffangboden 6 beispielsweise ringförmig, oval oder im Wesentlichen dreieckig ausgebildet sein. Von dem Auffangboden 6 aus erstrecken sich die Klemmarme 4 in Axialrichtung zu der offenen Stirnseite hin. Die Klemmarme 4 erstrecken sich von der radialen Außenkante des Auffangbodens 6 aus in Axialrichtung. Vorzugsweise ist der Auffangboden 6 aus einem Metall, beispielsweise aus einem Federstahl, wie dem Werkstoff 1.4310, gefertigt. Beispielsweise können die Klemmarme 4 über einen spitzwinkligen Eckverbinder/einen spitzwinkligen Eckverbindungsbereich/eine Biegestelle an dem Auffangboden 6 angebracht sein, so dass der Neigungswinkel an dem Eckverbinder zwischen den Klemmarmen 4 und dem Auffangboden 6 durch das Einstecken des Werkzeugs entgegen der Elastizität der Klemmarme 4 vergrößert wird. Insbesondere kann der Auffangboden 6 integral/werkstoffeinstückig mit den Klemmarmen 4 verbunden sein.

Der Auffangboden 6 ist bei einer unmittelbaren stoffschlüssigen Befestigung der Aufnahme-Expanderhülse 2 an der Halterungsvorrichtung 50 durch eine ebene, abkantfreie Platte gebildet, von der sich ausschließlich die Klemmarme 4 axial, d.h. in einer Richtung senkrecht zu der Platte, wegerstrecken und die in einem Mittenbereich an ihrer einen oder beiden Flachseiten einen Anschweiß-, Löt- oder Klebabschnitt 20 ausbildet. Mit anderen Worten besteht die Aufnahme-Expanderhülse 2 aus dem ebenen, abkantfreien Auffangboden 6, von dem sich die mehreren, in der dargestellten Ausführungsform drei, Klemmarme 4 axial in Richtung zu der offenen Stirnseite hin erstrecken. Somit erstrecken sich von der radialen Außenkante des Auffangbodens 6 ausschließlich die Klemmarme 4 in Axialrichtung.

Die Unterseite 16 des Auffangbodens 6 ist die von den Klemmarmen 4 abgewandte Axialseite des Auffangsbodens 6, d.h. eine axiale Außenfläche der Aufnahme-Expanderhülse 2. Die Oberseite 18 des Auffangbodens 6 ist die den Klemmarmen 4 zugewandte Axialseite des Auffangsbodens 6, d.h. eine (zwischen den Klemmarmen 4 angeordnete) axiale Innenfläche der Aufnahme-Expanderhülse 2. Die Oberseite 18 des Auffangbodens 6 bildet eine axiale Anschlagsfläche für das einzusteckende Werkzeug. Die Unterseite 16 und/oder die Oberseite 18 des Auffangbodens 6 besitzen oder bilden den vorzugsweise metallischen Anschweiß-, Löt-oder Klebabschnitt 20 aus, der zum unmittelbaren stoffschlüssigen, insbesondere (punkt-)schweißenden, Befestigen der Aufnahme-Expanderhülse 2 an der Halterungsvorrichtung 50, insbesondere der zweiten Lochplatte 54, angepasst und vorgesehen ist. Bei einer Ausbildung des Anschweiß-, Löt- oder Klebabschnitts 20 an der Unterseite 16 kann die Aufnahme-Expanderhülse 2 axial von oben (in der Einsteckrichtung des einzusteckenden Werkzeugs) auf die Halterungsvorrichtung 50 aufgesetzt/aufgelegt und stoffschlüssig daran befestigt werden. Bei einer Ausbildung des Anschweiß-, Löt- oder Klebabschnitts 20 an der Oberseite 18 kann die Aufnahme-Expanderhülse 2 axial von unten (entgegen der Einsteckrichtung des einzusteckenden Werkzeugs) auf die Halterungsvorrichtung 50 aufgesteckt und stoffschlüssig daran befestigt werden. Mit anderen Worten ist die Unterseite 16 und/oder die Oberseite 18 des Auffangbodens 6 an der Halterungsvorrichtung 50 angeschweißt oder anschweißbar.

Die Aufnahme-Expanderhülse 2 kann derart angepasst und vorgesehen sein, dass sie auf die zweite Lochplatte 54 der Halterungsvorrichtung 50 entgegen einer Einsteckrichtung des einzusteckenden Werkzeugs aufsteckbar ist, so dass die Klemmarme 4 durch die zweiten Löcher 62 hindurchgreifen und der Auffangboden 6 axial von der zweiten Lochplatte 54 als Abstandshalter hervorsteht. Mit anderen Worten kann die zweite Lochplatte 54 dadurch beispielsweise gegenüber einem (nicht dargestellten) Aufnahmekasten, in den die zweite Lochplatte 54 in der Einsteckrichtung des Werkzeugs eingesetzt wird, beabstandet gehalten werden. Das heißt, dass eine Unterseite der zweiten Lochplatte 54 um die Dicke des Auffangbodens 6 von dem Aufnahmekasten axial beabstandet gehalten werden kann. Gemäß einer Ausführungsform kann der Auffangboden 6 einen schweißbaren Einsatz oder eine schweißbare Beschichtung aufweisen.

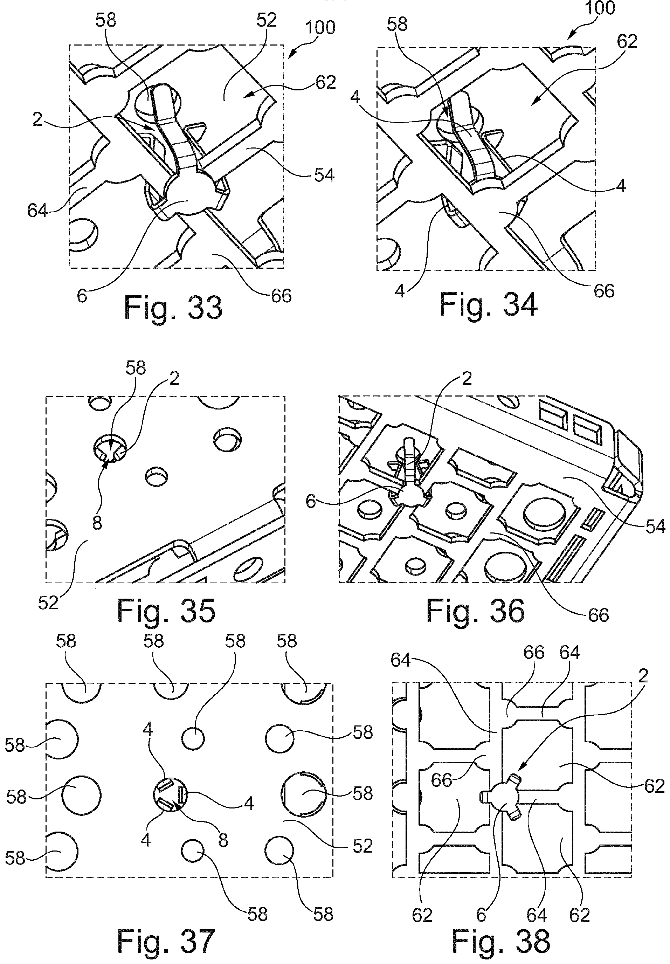

Fign. 33 bis 38 zeigen verschiedene Ansichten des Werkzeugsets 100 der dritten Ausführungsform, bei dem die Aufnahme-Expanderhülse 2 an der Halterungsvorrichtung 50 befestigt ist. In einer in Fign. 33, 36 und 38 gezeigten Weiterbildung der dritten Ausführungsform ist die Oberseite 18 des Auffangbodens 6 an der Halterungsvorrichtung 50, insbesondere an einem der Kreuzungspunkte 66 der zweiten Lochplatte 54 befestigt, vorzugsweise (durch Punktschweißen) angeschweißt. In einer in Fig. 34 gezeigten Weiterbildung der dritten Ausführungsform ist die Unterseite 16 des Auffangbodens 6 an der Halterungsvorrichtung 50, insbesondere an einem der Kreuzungspunkte 66 der zweiten Lochplatte 54 befestigt, vorzugsweise (durch Punktschweißen) angeschweißt.

In der in Fig. 34 gezeigten Weiterbildung ist die Aufnahme-Expanderhülse 2 (von oben, d.h. in Einsteckrichtung des Werkzeugs) auf die zweite Lochplatte 54 aufgesetzt. Das heißt, dass die Aufnahme-Expanderhülse 2 vollständig zwischen den beiden Lochplatten 52, 54 angeordnet sein kann. Der Auffangboden 6 und die durch den Kreuzungspunkt 66 gebildete Kreisfläche sind zentriert zueinander ausgerichtet. Die Unterseite/axiale Außenfläche 16 der Aufnahme-Expanderhülse 2 ist an der der ersten Lochplatte 52 zugewandte Axialseite der zweiten Lochplatte 54 angebracht, insbesondere durch Punktschweißen.

In der in Fign. 33, 36 und 38 gezeigten Weiterbildung ist die Aufnahme-Expanderhülse 2 (von unten, d.h. entgegen der Einsteckrichtung des Werkzeugs) auf die zweite Lochplatte 54 aufgesteckt bzw. in die zweite Lochplatte 54 eingesteckt. Der Auffangboden 6 und die durch den Kreuzungspunkt 66 gebildete Kreisfläche sind zentriert zueinander ausgerichtet. Die Oberseite/axiale Innenfläche 18 der Aufnahme-Expanderhülse 2 ist an der der ersten Lochplatte 52 abgewandten Axialseite der zweiten Lochplatte 54 angebracht, insbesondere durch Punktschweißen. Mit anderen Worten durchgreift jeder der Klemmarme 4 eines der zweiten Löcher 62 axial. Das heißt, dass die Aufnahme-Expanderhülse 2 axial an der der ersten Lochplatte 52 abgewandten Axialseite der zweiten Lochplatte 54 um die Dicke des Auffangbodens 6 hervorsteht. Dies hat den Vorteil, dass der Auffangboden 6 als ein Abstandshalter zwischen der zweiten Lochplatte 54 und dem (nicht dargestellten) Aufnahmekasten dienen kann.

Durch die fluchtende Anordnung der ersten Löcher 58 mit den Kreuzungspunkten 66 der zweiten Lochplatte fluchtet die durch die Klemmarme 4 gebildete Einstecköffnung 8 mit den ersten Löchern 58. So kann das einzusteckende Werkzeug in der Einsteckrichtung durch die erste Lochplatte 52 axial hindurchgesteckt (bis zu dem Auffangboden 6 bzw. der zweiten Lochplatte 54) und von den Eingriffsabschnitten 10 der Aufnahme-Expanderhülse 2 radial gehalten werden.

## Patentansprüche

1. Werkzeugset (100) zur steckgelagerten Aufnahme chirurgischer Werkzeuge, mit
einer Aufnahme-Expanderhülse (2) zur steckgelagerten Aufnahme chirurgischer Werkzeuge, die eine Anzahl von umfangsbeabstandeten, elastisch verformbaren Klemmarmen (4) und einen Auffangboden (6) aufweist, wobei die Klemmarme (4) an einer offenen Stirnseite der Aufnahme-Expanderhülse eine Einstecköffnung (8) für das Einstecken eines Werkzeugs ausbilden und radial nach innen ragende Eingriffsabschnitte (10) zur kraft- und/oder formschlüssigen Kontaktierung des Werkzeugs für das Halten des Werkzeugs haben, der Auffangboden (6) an einem der Einstecköffnung (8) gegenüberliegenden Endabschnitt der Aufnahme-Expanderhülse (2) ausgebildet ist, sich die Klemmarme (4) von dem Auffangboden (6) aus in Axialrichtung zu der offenen Stirnseite hin erstrecken, und der Auffangboden (6) durch eine vorzugsweise ebene, abkantfreie Platte gebildet ist, von der sich ausschließlich die Klemmarme (4) axial wegerstrecken und die in einem Mittenbereich an ihrer einen oder beiden Flachseiten einen Anschweiß-, Löt- oder Klebabschnitt ausbildet, und
einer Halterungsvorrichtung (50), die eine erste Lochplatte (52) mit ersten vorzugsweise runden Löchern (58), die durch erste Stege (60) voneinander getrennt sind, zum Einstecken eines Werkzeugs sowie eine parallel dazu beabstandete, mit der ersten Lochplatte (52) verbundene zweite Lochplatte (54) mit voneinander durch zweite Stege (64) getrennten zweiten Löchern (62) aufweist,
**dadurch gekennzeichnet, dass**
sich die zweiten Stege (64) an mit den ersten Löchern fluchtend angeordneten Kreuzungspunkten (66) überschneiden und der Auffangboden (6) der Aufnahme-Expanderhülse (2) an den Kreuzungspunkten (66) der zweiten Stege (64) stoffschlüssig befestigt oder befestigbar, insbesondere angeschweißt oder anschweißbar ist.

2. Werkzeugset (100) nach Anspruch 1, **dadurch gekennzeichnet, dass** die zweiten Stege (64) an den Kreuzungspunkten (66) eine Schweiß-, Löt- oder Klebfläche bilden, die zumindest so groß wie eine Fläche des Auffangbodens (6), insbesondere wie der Anschweiß-, Löt- oder Klebabschnitt ist.

3. Werkzeugset (100) nach Anspruch 1 oder 2, **dadurch gekennzeichnet, dass** die zweiten Löcher (62) im Wesentlichen rechteckig, insbesondere quadratisch ausgebildet und in 60° versetzten Reihen angeordnet sind, und sich die zweiten Stege (64) derart verbreitern, dass sie sich an den Eckpunkten der zweiten Löcher (62) vorzugsweise konvex in Richtung zur Lochmitte hin wölben.

4. Werkzeugset (100) nach einem der Ansprüche 1 bis 3, **dadurch gekennzeichnet, dass** die Unterseite (16) des Auffangbodens (6) an einer der ersten Lochplatte (52) zugewandten Axialseite der zweiten Lochplatte (54) stoffschlüssig befestigt oder befestigbar, insbesondere angeschweißt oder anschweißbar ist und/oder dass die Oberseite (18) des Auffangbodens (6) an einer der ersten Lochplatte (52) angewandten Axialseite der zweiten Lochplatte (54) stoffschlüssig befestigt oder befestigbar, insbesondere angeschweißt oder anschweißbar ist.

5. Werkzeugset (100) nach Anspruch 4, **dadurch gekennzeichnet, dass** die Aufnahme-Expanderhülse (2), mit ihren Klemmarmen (4) durch die zweiten Löcher (62) hindurchgreifend, entgegen der Einsteckrichtung des einzusteckenden Werkzeugs auf einen der Kreuzungspunkte (66) so aufsteckbar ist, dass der Auffangboden (6) entgegen der Einsteckrichtung des einzusteckenden Werkzeugs von der zweiten Lochplatte (54) als axialer Abstandshalter hervorsteht.

6. Werkzeugset (100) nach einem der Ansprüche 1 bis 5, **dadurch gekennzeichnet, dass** die ersten Löcher (58) der ersten Lochplatte (52) in parallel beabstandeten Reihen angeordnet sind und die Lochmitten jeweils zwei benachbarter Reihen um eine Stegbreite der zweiten Stege (64) zueinander versetzt angeordnet sind.

7. Werkzeugset (100) nach einem der Ansprüche 1 bis 6, **dadurch gekennzeichnet, dass** der Auffangboden (6) einen schweißbaren Einsatz oder eine schweißbare Beschichtung aufweist.

## Claims

1. A tool set (100) for plug-in holding of surgical tools, comprising
an expander holder (2) for plug-in holding of surgical tools, comprising a number of circumferentially spaced, elastically deformable clamping arms (4) and a catching base (6), wherein the clamping arms (4) form an insertion opening (8) for the insertion of a tool on an open front side of the expander holder and have radially inward projecting engagement portions (10) for the force-fitting and/or form-fitting contacting of the tool for holding the tool, the catching base (6) being formed at an end portion of the expander holder (2) opposite to the insertion opening (8) and the clamping arms (4) extend from the catching base (6) in the axial direction toward the open front side, and the catching base (6) is formed by a preferably flat, chamfer-free plate from which exclusively the clamping arms (4) extend axially and the plate forms a welding, soldering or gluing portion in a central region on its one or both flat sides, and
a retaining device (50) having a first perforated plate (52) with first preferably round holes (58) separated by first crosspieces (60) for insertion of a tool and a second perforated plate (54) spaced parallel thereto and connected to the first perforated plate (52) and having second holes (62) separated from each other by second crosspieces (64),
**characterized in that**
the second crosspieces (64) intersect at junction points (66) arranged in alignment with the first holes, and the catching base (6) of the expander holder (2) is firmly bonded or bondable, in particular welded or weldable, to the junction points (66) of the second crosspieces (64).

2. The tool set (100) according to claim 1, **characterized in that** the second crosspieces (64) at the junction points (66) form a welding, soldering or gluing surface which is at least as large as an area of the catching base (6), in particular as the welding, soldering or gluing portion.

3. The tool set (100) according to claim 1 or 2, **characterized in that** the second holes (62) are essentially rectangular, in particular square, and are arranged in rows offset by 60°, and the second crosspieces (64) widen in such a way that they curve at the corner points of the second holes (62), preferably convexly, in the direction of the hole center.

4. The tool set (100) according to one of the claims 1 to 3, **characterized in that** the lower side (16) of the catching base (6) is firmly bonded or bondable, in particular welded or weldable, to an axial side of the second perforated plate (54) facing the first perforated plate (52), and/or **in that** the upper side (18) of the catching base (6) is firmly bonded or bondable, in particular welded or weldable, to an axial side of the second perforated plate (54) facing the first perforated plate (52).

5. The tool set (100) according to claim 4, **characterized in that** the expander holder (2), passing with its clamping arms (4) through the second holes (62), can be placed on one of the junction points (66) against the insertion direction of the tool to be inserted in such a way that the catching base (6) projects from the second perforated plate (54) as an axial spacer against the insertion direction of the tool to be inserted.

6. The tool set (100) according to any one of claims 1 to 5, **characterized in that** the first holes (58) of the first perforated plate (52) are arranged in parallel spaced rows and the hole centers of each two adjacent rows are arranged offset from each other by a crosspiece width of the second crosspieces (64).

7. The tool set (100) according to any one of claims 1 to 6, **characterized in that** the catching base (6) has a weldable insert or a weldable coating.

## Revendications

1. Jeu d'instruments (100) pour la réception enfichée d'instruments chirurgicaux, avec une douille expansible de réception (2) pour la réception enfichée d'instruments chirurgicaux, qui présente un certain nombre de bras de serrage (4) espacés sur la circonférence et déformables élastiquement et un fond de réception (6), dans lequel les bras de serrage (4) réalisent, sur un côté avant ouvert de la douille de réception-expansion, une ouverture d'enfichage (8) pour l'enfichage d'un instrument et présentent des sections d'engagement (10) faisant saillie radialement vers l'intérieur pour la mise en contact par adhérence et/ou par complémentarité de forme de l'instrument pour le maintien de l'outil, le fond de réception (6) est formé sur une section d'extrémité de la douille expansible de réception (2) opposée à l'ouverture d'enfichage (8), les bras de serrage (4) s'étendent à partir du fond de réception (6) en direction axiale vers le côté avant ouvert, et le fond de réception (6) est formé par une plaque de préférence plane, sans arêtes, à partir de laquelle seuls les bras de serrage (4) s'étendent axialement et qui réalise dans une zone centrale, sur l'un de ses un ou deux côtés plats, une section de soudage, de brasage ou de collage, et un dispositif de fixation (50) qui présente une première plaque perforée (52) avec de premiers trous (58), de préférence ronds, qui sont séparés les uns des autres par de premières nervures (60), pour l'insertion d'un instrument, ainsi qu'une seconde plaque perforée (54) espacée parallèlement à la première et reliée à la première plaque perforée (52), avec des seconds trous (62) séparés les uns des autres par de secondes nervures (64),
**caractérisé en ce que**
les secondes nervures (64) se recoupent en des points d'intersection (66) agencés en alignement avec les premiers trous et le fond de réception (6) de la douille expansible de réception (2) est fixé ou peut être fixé, en particulier est soudé ou peut être soudé, par complémentarité de matière aux points de croisement (66) des secondes nervures (64).

2. Jeu d'instruments (100) selon la revendication 1, **caractérisé en ce que** les secondes nervures (64) forment, aux points d'intersection (66), une surface de soudage, de brasage ou de collage qui est au moins aussi grande qu'une surface du fond de réception (6), en particulier que la section de soudage, de brasage ou de collage.

3. Jeu d'instruments (100) selon la revendication 1 ou 2, **caractérisé en ce que** les seconds trous (62) sont sensiblement rectangulaires, en particulier carrés, et sont agencés en rangées décalées de 60°, et les secondes nervures (64) s'élargissent de sorte qu'elles se courbent aux points d'angle des seconds trous (62), de préférence de manière convexe, en direction du centre du trou.

4. Jeu d'instruments (100) selon l'une quelconque des revendications 1 à 3, **caractérisé en ce que** le côté inférieur (16) du fond de réception (6) est fixé ou peut être fixé par complémentarité de matière sur un côté axial de la seconde plaque perforée (54) orienté vers la première plaque perforée (52), en particulier être soudé ou peut être soudé et/ou **en ce que** le côté supérieur (18) du fond de réception (6) est fixé ou peut être fixé, en particulier être soudé ou peut être soudé, par complémentarité de matière sur un côté axial de la seconde plaque perforée (54) orienté vers la première plaque perforée (52).

5. Jeu d'instruments (100) selon la revendication 4, **caractérisé en ce que** la douille expansible de réception (2), en passant avec ses bras de serrage (4) à travers les seconds trous (62), peut être enfichée sur l'un des points d'intersection (66) dans le sens contraire au sens d'enfichage de l'instrument à insérer, de sorte que le fond de réception (6) fasse saillie depuis la seconde plaque perforée (54) en tant que nervure axiale dans le sens contraire au sens d'enfichage de l'instrument à insérer.

6. Jeu d'instruments (100) selon l'une quelconque des revendications 1 à 5, **caractérisé en ce que** les premiers trous (58) de la première plaque perforée (52) sont agencés en rangées parallèles espacées et les centres des trous de respectivement deux rangées voisines sont décalés les uns par rapport aux autres d'une largeur de nervure des secondes nervures (64).

7. Jeu d'instruments (100) selon l'une quelconque des revendications 1 à 6, **caractérisé en ce que** le fond de réception (6) présente un insert ou un revêtement pouvant être soudé.
